# EUROPÄISCHE PATENTSCHRIFT

(11) **EP 1 280 472 B1**
(45) Veröffentlichungstag und Bekanntmachung des Hinweises auf die Patenterteilung: **02.11.2006**
(21) Anmeldenummer: 01940457.3
(22) Anmeldetag: 11.05.2001
(51) Int. Cl.: A61B 19/00, A61C 19/04

(54) **SENSORANORDNUNG ZUR ERFASSUNG VON LAGE- UND POSITIONSVERÄNDERUNGEN EINES PROBANDEN IN EINEM NEURONAVIGATIONSSYSTEM**
SENSOR DEVICE FOR DETECTING THE CHANGES IN POSITION AND LOCATION OF A PROBAND IN A NEURONAVIGATION SYSTEM
DISPOSITIF DE DETECTION DE MODIFICATIONS DE LOCALISATION ET DE POSITION D'UN PATIENT DANS UN SYSTEME DE NEURONAVIGATION

(30) Priorität: 11.05.2000 DE 10022937
(43) Veröffentlichungstag der Anmeldung: 05.02.2003
(73) Patentinhaber: Schaerer Mayfield USA, Inc., Cincinnati, OH 45227 (US)
(72) Erfinder: WARSCHEWSKE, Udo, 12249 Berlin (DE); SCHLEICH, Arno, 12207 Berlin (DE)
(74) Vertreter: Schmidt, Steffen J.
(86) Internationale Anmeldenummer: PCT/EP2001/005402
(87) Internationale Veröffentlichungsnummer: WO 2001/085046

(56) Entgegenhaltungen:
- EP-A- 0 826 345
- EP-A- 0 904 733
- WO-A-96/08209
- WO-A-98/27892
- DE-A- 19 715 202
- US-A- 5 954 647

## Beschreibung

Die Erfindung betrifft eine Sensoranordnung zur Erfassung von Lage- und Positionsveränderungen eines Probanden in einem Neuronavigationssystem, welches zur Durchführung und Unterstützung von chirurgischen Eingriffen dient, wobei die Anordnung am Kopf des Probanden fixierbar ist und die Ausgangssignale als Korrekturdaten einem Systemrechner zugeführt sind, gemäß Oberbegriff des Patentanspruchs 1.

Aus der PCT WO96/08209 ist ein Neuronavigationssystem sowie eine Tracking-Einrichtung für medizinische Anwendungen vorbekannt. Dort ist eine Bilddatenbank für präoperativ angefertigte Kernspin- oder Computertomographieaufnahmen vorhanden und ein Personal-Computer oder Steuerrechner steht mit einem Monitor zur Bilddatenverarbeitung und -darstellung in Verbindung. Eine Tracking-Einrichtung dient dem Bestimmen der momentanen Position eines Instruments und Ableiten von Darstellungen der Patientenanatomie anhand der in der Bilddatenbank abgelegten Aufnahmen. Weiterhin sind dort Mittel zum Extrahieren von anatomischen Strukturen aus den Rohdatensätzen der präoperativen Aufnahmen und Bereitstellen dieser Strukturen in Form von visualisierbaren 3D-Bilddatensätzen vorgesehen. Ein Transmitter erzeugt ein magnetisches Feld in der Navigationsumgebung, wobei das Zeigernavigationsinstrument einen Magnetfeldsensor besitzt. Die Position des Instruments kann durch eine entsprechende Feldstärkebestimmung ermittelt werden.

Zur Bestimmung einer Bezugslage ist ein weiterer Sensor vorgesehen, welcher mit Hilfe einer Klammer am Kopf des Patienten im Bereich der Nase fixiert wird. Eine solche Befestigungsart für einen Korrektursensor, der Lageveränderungen des Kopfes des Probanden aufnimmt, ist jedoch unzuverlässig und stört insbesondere dann, wenn neurochirurgische Eingriffe oder chirurgische Maßnahmen im Hals-Nasen-Ohrenbereich vorgesehen sind.

Bei einer älteren, auf die Anmelderin zurückgehenden Lehre wird bei einem Navigationssystem zur Durchführung und Unterstützung von chirurgischen Eingriffen auf einen speziellen Gleichfeld-Transmitter zurückgegriffen, wobei im Zeigernavigationsinstrument ein integraler Magnetfeldsensor vorgesehen ist. Der Transmitter zum Erzeugen des magnetischen Gleichfelds der Tracking-Einrichtung ist dort am Operationstisch oder einer dort vorgesehenen Kopfauflage, jedoch außerhalb des Operationsfelds angeordnet, wodurch sich eine feste, reproduzierbare Lagebeziehung zwischen dem zu navigierenden Organ des Patienten und dem Transmitter unabhängig von der Lage des Operationstisches im Raum ergibt. Der Magnetfeldsensor gemäß der bereits vorgeschlagenen Lehre liefert Signale zum Ableiten von Position und/oder Bewegungsrichtung des Zeigernavigationsinstruments anhand des definierten magnetischen Gleichfelds und dessen Feldorientierung, wobei diese Signale sowohl auf einem PC-Monitor darstellbar sind, als auch zur Steuerung der Nachlade- und Aktualisierungsvorgänge eines Bildbearbeitungsmoduls dienen.

Ebenfalls wurde dort vorgeschlagen, einen weiteren Magnetfeldsensor anzuordnen, wobei dieser unmittelbar am Probanden, bevorzugt am Kopf befestigbar ist, um Lage- und Positionsveränderungen bezogen auf den Gleichfeld-Transmitter zu erfassen. Die Ausgangssignale dieses zweiten Magnetfeldsensors werden als Korrekturdaten dem Steuerrechner zugeführt, um ein quasi dynamisches oder bezogen auf die Ursprungsänderung veränderliches Koordinatensystem festzulegen.

Es hat sich jedoch gezeigt, daß die Befestigung des weiteren Sensors am Kopf eines Probanden bzw. Patienten nicht unproblematisch ist, da ein sicheres Fixieren erforderlich wird und andererseits aber der Operateur in seiner Tätigkeit durch diesen weiteren Sensor nicht behindert werden darf.

Aus dem gattungsbildenden Stand der Technik gemäß US 5,954,647 ist eine Sensoranordnung nach dem Oberbegriff von Patentanspruch 1 bekannt. Diese umfasst eine Zahnschiene, bei der am vorderen. Bereich, das heißt an dem mit den Schneidezähnen zu koppelnden Bereich, eine Sensoranordnung anbringbar ist, die auf eine Befestigungsplatte an der Zahnschiene aufsteckbar ist. Diese Anordnung hat den Nachteil, dass sie die Öffnung der Mundhöhle zumindest teilweise blockiert und auch vom Gesicht des Patienten im Gebrauchszustand nach vorne absteht. Dadurch kann die Arbeit eines Operateurs behindert werden.

Aus dem Dokument EP 0 826 345 A1 ist ferner eine Sensoranordnung bekannt, die über eine U-profilartige Zahnschiene an einer Zahnreihe eines Patienten anbringbar ist. Auch bei dieser Anordnung ist die Sensoranordnung derart mit der U-Profilschiene gekoppelt, dass sie sich zumindest teilweise in der Mundhöhle des Patienten erstreckt sowie aus dem Gesichtsfeld vorsteht, so dass sie die Arbeit des Operateurs behindern kann.

Aus dem Vorgenannten ist es daher Aufgabe der Erfindung, eine Sensoranordnung zur Erfassung von Lage- und Positionsveränderungen eines Probanden in einem Neuronavigationssystem anzugeben, welches der Durchführung und Unterstützung von chirurgischen Eingriffen dient. Die zu schaffende Anordnung soll sicher am Kopf des Probanden fixierbar sein, ohne dass der Operateur in seiner Arbeit behindert ist. Darüber hinaus muss die Sensoranordnung präoperativ leicht und ohne Aufwand angebracht werden können und darf bezüglich der Befestigungsart nicht zu unnötig hohen Kosten oder Aufwendungen führen.

Die Lösung der Aufgabe der Erfindung erfolgt mit einer Sensoranordnung gemäss den Merkmalen des Patentanspruchs 1, wobei die Unteransprüche mindestens zweckmäßige Ausgestaltungen und Weiterbildungen umfassen.

Der Grundgedanke der Erfindung liegt demnach in einer zweiteiligen Sensoranordnung, die einerseits eine profilierte Zahnschiene und andererseits eine Sensorkapsel umfasst. Zahnschiene und Sensorkapsel sind mit Mitteln zum lösbaren gegenseitigen Halten versehen. Die Zahnschiene selbst dient der Aufnahme einer Befestigungshaftmasse, z. B. einer üblichen Zweikomponenten-Zahnabdruckmasse. Mit Hilfe dieser Haftmasse wird die Zahnschiene an einer Zahnreihe des Patienten im Mundraum fixiert.

Die Sensorkapsel kann bevorzugt mittels einer Prismen-, insbesondere Flach- oder Schwalbenschwanzführung mit einem Abschnitt der profilierten Zahnschiene verbunden werden.

Selbstverständlich besteht auch die Möglichkeit, die Sensorkapsel in üblicher Weise durch Schrauben oder in ähnlicher Art kraftschlüssig an der profilierten Zahnschiene zu befestigen.

Die Sensorkapsel selbst besitzt bei einem Ausführungsbeispiel eine im wesentlichen rechteckige Gehäuseform, wobei an der Gehäuselängsseite das zur Zahnschiene komplementäre Führungsmittel ausgebildet und weiterhin am Gehäuse eine Befestigungseinrichtung für ein Sensorkabel vorgesehen ist. Diese Befestigungseinrichtung kann eine feuchtigkeitsgeschützte Steckverbindung sein, die mit einer zugehörigen Steckeinrichtung am Kabelende wechselwirkt.

Die Zahnschiene weist bevorzugt eine leicht gekrümmte Form auf, die im wesentlichen einem Abschnitt einer Zahnreihe entspricht. Die Fläche zwischen Innenkreis und Außenkreis besitzt Durchbrüche oder Aussparungen, die dem Fixieren der Befestigungshaftmasse dienen, so daß ein Verrutschen der an der Zahnreihe befestigten Zahnschiene ausgeschlossen wird.

Das Profil der Zahnschiene ist bei einem Ausführungsbeispiel U-förmig ausgebildet, wobei ein Profilschenkel eine erste geringere Dicke und der weitere Profilschenkel eine zweite, größere Dicke aufweist. Der Profilschenkel größerer Dicke dient der Aufnahme des Mittels zum lösbaren Halten der Sensorkapsel. Zum Beispiel kann in diesem Profilschenkel mit größerer Dicke eine Nut zur Aufnahme einer Schwalbenschwanz- oder Flachführung, d.h. eines entsprechend am Sensorkapselgehäuse geformten Steges eingebracht sein.

Im Gebrauchszustand wird die Anordnung im Mundraum des Probanden mittels der vorerwähnten Haftmasse an einer Zahnreihe befestigt, wobei der Profilschenkel größerer Dicke mit der dort angebrachten Sensorkapsel zur Außenseite der Zahnreihe in Wangenrichtung orientiert ist. Auf diese Weise ist sichergestellt, daß die Mundhöhle des Probanden im wesentlichen frei bleibt, so daß hier keinerlei Einschränkungen für den Chirurgen gegeben sind. Insbesondere kommt es zu keinen Behinderungen mit anderen medizinischen Gerätschaften, z.B. einem Tubus der Anästhesie. Die unmittelbare Nähe der Anordnung zum Operationsfeld sichert weiterhin ein hohes Maß an Genauigkeit durch Minimierung der Fehlervektoren.

Die Bildregistrierung für die Neuronavigation kann bereits bei freier Kopfbewegung des Probanden ohne weitere Mittel als die beschriebene Sensoranordnung erfolgen.

Die von der Sensoranordnung erhaltenen Daten, z.B. Magnetfelddaten, werden bei der Berechnung der Position des eigentlichen Neuronavigations-Zeigerinstruments entsprechend berücksichtigt und einem im System vorhandenen Rechner zugeführt.

Selbstverständlich sind abweichend von der beschriebenen Flach-, Prismen- oder Schwalbenschwanzführung auch rastende, d.h. sogenannte Snap-in-Verbindungen zwischen Sensorkapsel und Zahnschiene denkbar, ohne daß das vorgestellte Prinzip der Erfindung verlassen wird.

Die Zahnschiene kann aus hygienischen Gründen als Einwegteil ausgebildet sein, wobei aber auch die Möglichkeit besteht, diese aus einem sterilisierbaren Material zum Zwecke der Mehrfachnutzung zu fertigen.

Die Erfindung soll nachstehend anhand eines Ausführungsbeispiels sowie unter Zuhilfenahme von Figuren näher erläutert werden.

Hierbei zeigen:

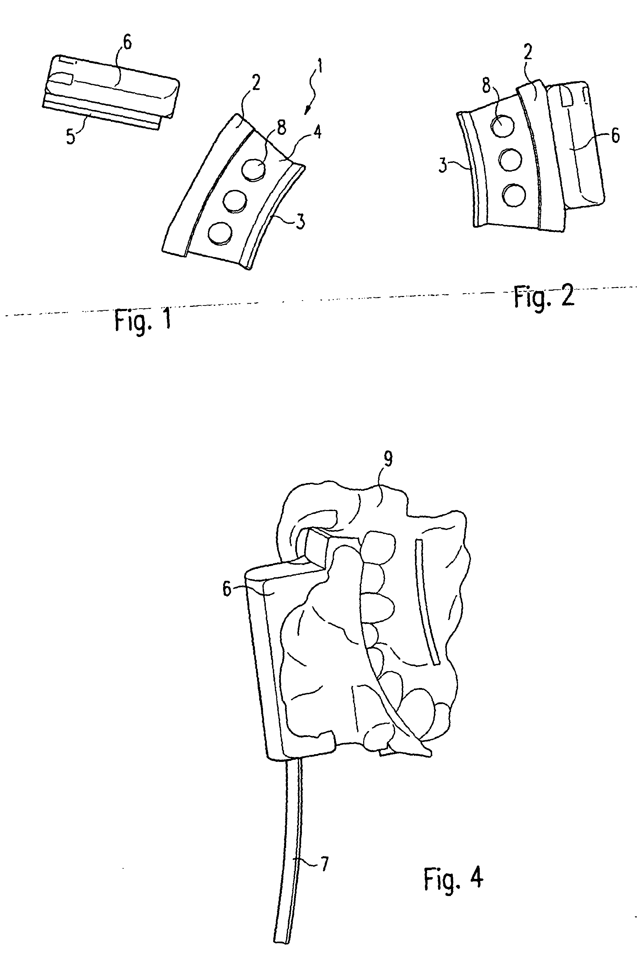

- Fig. 1: eine Ansicht von Sensorkapsel und profilierter Zahnschiene vor dem Verbinden;
- Fig. 2: eine Ansicht einer Sensorkapsel, die mit der Zahnschiene über eine geeignete Führung rastend verbunden ist;
- Fig. 3: eine im Mundraum eines Probanden eingesetzte Sensoranordnung; und
- Fig. 4: eine Darstellung der Sensoranordnung mit Befestigungshaftmasse nach Entfernen aus dem Mundraum des Probanden.

Gemäß den Figuren umfaßt die Sensoranordnung eine profilierte Zahnschiene 1 mit einem Profilschenkel größerer Dicke 2 und einem Profilschenkel kleinerer Dicke 3. Der die Schenkel verbindende Abschnitt 4 weist beim gezeigten Ausführungsbeispiel kreisförmige Durchbrüche 8 oder Bohrungen auf, die der besseren Aufnahme der Befestigungshaftmasse 9 dienen, so daß ein unerwünschtes Verschieben der Anordnung ausgeschlossen wird.

Im Bereich des Schenkels größerer Dicke 2 ist eine Längsnut eingebracht, die komplementär zu einem Führungsvorsprung 5 am Gehäuse 6 der Sensorkapsel ausgebildet ist.

Beispielsweise kann die Führung als Flach-, Prismen- oder Schwalbenschwanzführung realisiert werden, wobei selbstverständlich der Führungsvorsprung 5 auch am Schenkel größerer Dicke 2 und die hierzu komplementäre Nut an der Sensorkapsel bzw. dem Gehäuse 6 ausführbar ist.

Am Gehäuse 6 ist eine nicht gezeigte Steckbuchse zum mechanischen Befestigen und elektrischen Kontaktieren eines Kabels 7 vorhanden.

Die Form des Gehäuses 6 ist beim Ausführungsbeispiel im wesentlichen rechteckig und langgestreckt, so daß die Sensorkapsel im Raum zwischen Zahnreihe und Wangeninnenbereich Platz findet.

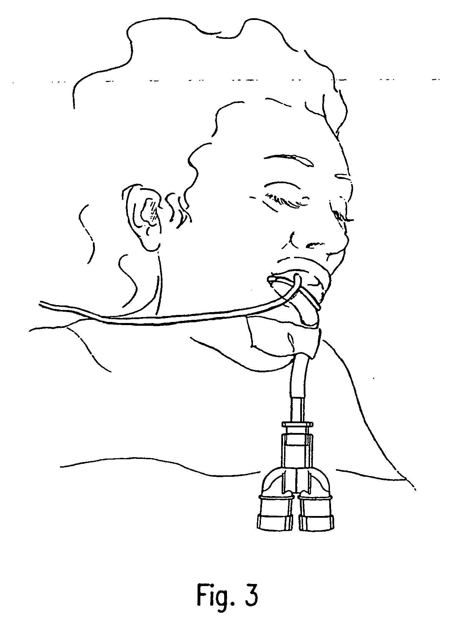

Die Art der Befestigung der Sensoranordnung ist in der Fig. 3 illustriert, wobei auch deutlich wird, daß die Mundhöhle frei bleibt, um z.B. einen Tubus aufzunehmen.

Die Zahnschiene 1 weist eine leicht gekrümmte Form auf, die dem Verlauf eines Abschnitts einer Zahnreihe entspricht.

Im Gebrauchszustand wird die Zahnschiene 1 mit Hilfe der Haftmasse 9 an einer Zahninnenseite des Probanden befestigt, wobei der Profilschenkel größerer Dicke 2 mit der dort angebrachten Sensorkapsel zur Außenseite der Zahnreihe in Wangenrichtung orientiert ist. Als Haftmasse 9 wird bevorzugt Zweikomponenten-Zahnabdruckmasse verwendet.

Die Zahnschiene, welche von der Sensorkapsel trennbar ist, kann als Einwegteil in besonders preiswerter Form gefertigt oder zur Mehrfachnutzung aus einem sterilisierbaren Material hergestellt werden.

Alles in allem gelingt es mit der vorgestellten Erfindung, den Patientenkopf beweglich zu halten, was im Vergleich zu einer bisher erforderlichen starren Fixierung operative Vorteile nach sich zieht. Darüber hinaus kann durch das einfache und sichere Fixieren des Sensors im Mundraum des Probanden eine Korrekturdatenerfassung erfolgen, so daß Lage- und Positionsveränderungen des Kopfes ohne weiteres online erfaßt und bei der Bestimmung der Lage des Zeigerinstruments berücksichtigt werden können.

Es liegt im Sinne der Erfindung, daß die Profilierung der Zahnschiene auch von einer U-Form abweichen kann, solange diese sicher haftend an einer Zahnreihe befestigbar ist. Das Anbringen des Sensoranordnung mit Hilfe der Zahnschiene und der schnell abbindenden Abdruckmasse geschieht zum Zeitpunkt der Patientenvorbereitung im Operationsraum nach der Entubation des Patienten.

### Bezugszeichenliste

- 1: Zahnschiene
- 2: Schenkel größerer Dicke
- 3: Schenkel kleinerer Dicke
- 4: Verbindungsabschnitt der Schenkel
- 5: Führungsvorsprung
- 6: Gehäuse
- 7: Kabel
- 8: Durchbrüche im Verbindungsabschnitt
- 9: Haftmasse

## Patentansprüche

1. Sensoranordnung zur Erfassung von Lage- und Positionsveränderungen eines Probanden in einem Neuronavigationssystem, welches der Durchführung und Unterstützung von chirurgischen Eingriffen dient, wobei die Anordnung am Kopf des Probanden fixierbar ist und die Ausgangssignale als Korrekturdaten einem Systemrechner zugeführt sind, umfassend eine gekrümmte Zahnschiene (1) zur Aufnahme einer Befestigungshaftmasse (9) und eine Sensorkapsel (6) mit Mitteln zum lösbaren Halten an der Zahnschiene, wobei die Fläche zwischen Innenkreis und Aussenkreis der gekrümmten Zahnschiene (1) Durchbrüche (8) oder Aussparungen zum Fixieren der Befestigungshaftmasse (9) umfasst,
**dadurch gekennzeichnet, dass** die Zahnschiene (1) ein im Wesentlichen U-förmiges Profil besitzt, wobei ein Profilschenkel (3) eine erste geringe Dicke und der weitere Profilschenkel (2) eine zweite größere Dicke aufweist und am Profilschenkel (2) größerer Dicke das Mittel zum lösbaren Halten der Sensorkapsel (6) ausgebildet ist, und dass die Zahnschiene (1) derart ausgebildet ist, dass sie im Gebrauchszustand im Mundraum des Probanden mittels der Haftmasse (9) so an einer Zahnreihe befestigt ist, dass der Profilschenkel (2) größerer Dicke mit der dort angebrachten Sensorkapsel (6) zur Außenseite der Zahnreihe in Wangenrichtung orientiert ist, um die Mundhöhle im wesentlichen frei zu halten.

2. Anordnung nach Anspruch 1,
**dadurch gekennzeichnet, daß**
die Mittel zum lösbaren Halten als Prismen-, insbesondere Flach- oder Schwalbenschwanzführung ausgebildet sind.

3. Anordnung nach Anspruch 1 oder 2,
**dadurch gekennzeichnet, daß**
die Sensorkapsel eine im wesentlichen rechteckige Gehäuseform aufweist, wobei an der Gehäuselängsseite das zur Zahnschiene komplementäre Führungsmittel ausgebildet und weiterhin am Gehäuse eine Befestigungseinrichtung für ein Sensorkabel vorgesehen ist.

4. Anordnung nach einem der vorangegangenen Ansprüche,
**dadurch gekennzeichnet, daß**
die Zahnschiene als Einwegteil ausgebildet oder zur Mehrfachnutzung aus einem sterilisierbaren Material gefertigt ist.

## Claims

1. Sensor arrangement for detecting changes in location and position of a patient in a neuronavigation system which serves to perform and support surgical interventions, wherein the arrangement can be fixed to the head of the patient and the output signals are supplied as correction data to a system computer, said sensor arrangement comprising a curved tooth channel (1) for receiving an adhesive fixing compound (9), and a sensor capsule (6) having means for detachably mounting on the tooth channel, wherein the area between the inner circle and the outer circle of the curved tooth channel (1) comprises through-holes (8) or cavities for fixing of the adhesive fixing compound (9),
**characterized in that** the tooth channel (1) has an essentially U-shaped profile, one profile limb (3) having a first, lesser, thickness, and the further profile limb (2) having a second, greater, thickness, and the means for detachably mounting the sensor capsule (6) being realized on the profile limb (2) of greater thickness, and the tooth channel (1) is so realized that, in its state of use, it is fixed in the oral cavity of the patient by means of the adhesive compound (9) in such a manner that the profile limb (2) of greater thickness, including the sensor capsule (6) mounted thereon, is oriented towards the outside of the row of teeth in the direction of the cheek, so as to keep the oral cavity essentially free.

2. Arrangement according to claim 1,
**characterized in that**
the means for detachable mounting are realized as a prismatic, in particular flat or dovetail guide.

3. Arrangement according to either of claims 1 or 2,
**characterized in that**
the sensor capsule has an essentially rectangular housing shape, the guide means complementary to the tooth channel being realized on the longitudinal side of the housing and, furthermore, an attachment means for a sensor cable being provided on the housing.

4. Arrangement according to any one of the preceding claims,
**characterized in that**
the tooth channel is realized as a disposable part or is made of a sterilizable material for multiple use.

## Revendications

1. Dispositif de détection des modifications de localisation et de position d'un patient dans un système de neuronavigation servant à effectuer et à assister des interventions chirurgicales, le dispositif de détection pouvant être fixé à la tête du patient et les signaux de sortie étant acheminés sous forme de données de correction vers un calculateur du système, comprenant une attelle dentaire courbe (1) destinée à recevoir une masse adhésive de fixation (9) et une capsule de détection (6) munie de moyens pour son maintien amovible sur l'attelle dentaire, la surface comprise entre le cercle intérieur et le cercle extérieur de ladite attelle dentaire courbe (1) présentant des perforations (8) ou des ouvertures pour la fixation de la masse adhésive de fixation (9),
**caractérisé en ce que** l'attelle dentaire (1) se présente pour l'essentiel sous la forme d'un profilé en U, une branche (3) du profilé affichant une première faible épaisseur et l'autre branche (2) du profilé une deuxième épaisseur plus importante, et le moyen destiné au maintien amovible de la capsule de détection (6) étant formé sur la branche (2) du profilé d'épaisseur plus importante, et **en ce que** l'attelle dentaire (1) est conçue de manière à être, à l'état monté dans la cavité buccale du patient, fixée à l'aide de la masse adhésive (9) à une rangée de dents de telle sorte que la branche du profilé (2) d'épaisseur plus importante supportant la capsule de détection (6) soit orientée vers le côté extérieur de la rangée des dents, en direction de la joue, pour que la cavité de la bouche reste pour l'essentiel dégagée.

2. Dispositif selon la revendication 1,
**caractérisé en ce que**
lesdits moyens de maintien amovible sont conçus sous forme de glissière prismatique, plus particulièrement de glissière plate ou de glissière à queue d'aronde.

3. Dispositif selon la revendication 1 ou 2,
**caractérisé en ce que**
la capsule de détection présente une forme de boîtier pour l'essentiel rectangulaire, des moyens de guidage complémentaires à l'attelle dentaire étant formés sur le côté longitudinal du boîtier et un dispositif destiné à la fixation d'un câble de détecteur étant prévu sur le boîtier.

4. Dispositif selon l'une des revendications précédentes,
**caractérisé en ce que**
l'attelle dentaire est conçue comme une pièce pour une utilisation unique ou fabriquée à partir d'une matière stérilisable pour des utilisations répétées.
